# EUROPEAN PATENT APPLICATION

(11) **EP 1 026 244 A1**
(43) Date of publication of application: **09.08.2000**
(21) Application number: 99102172.6
(22) Date of filing: 03.02.1999
(51) Int. Cl.: C12N 15/12, C12N 15/62, C07K 14/46, A61K 38/17, G01N 33/50, G01N 33/68, C12Q 1/68

(54) **Proteins Capable of Triggering G2/M transition and of Interacting with CDC2- and Cyclin B**

(71) Applicant: EUROPÄISCHES LABORATORIUM FÜR MOLEKULARBIOLOGIE (EMBL), D-69117 Heidelberg (DE)
(72) Inventor: The designation of the inventor has not yet been filed
(74) Representative: Böhm, Brigitte, Dipl.-Chem. Dr.

(57) **Abstract**

A DNA sequence according to the invention contains (a) a sequence as shown in SEQ ID NO.1 or 2, (b) a sequence which encodes the same protein as (a) but is degenerate as a result of the genetic code, (c) a sequence hybridizing under stringent conditions to the sequences of (a) and/or (b), (d) a genomic sequence containing the sequence of (a), (b) or (c) and further containing one or more introns, or (e) a sequence which differs from (a), (b), (c) or (d) due to its origin from a different species. A protein according to the invention is encoded by such DNA sequence and can be used for inducing oocyte maturation and/or promoting cell division and/or differentiation and/or proliferation, in a pharmaceutical compostion or as diagnostic agent.

## Description

The present invention relates to DNA sequences, expression vectors containing such DNA sequences, proteins encoded thereby, the use of these proteins for inducing oocyte maturation or promoting cell division and in a pharmaceutical composition, uses as diagnostic markers or for identifying substances blocking the cell cycle progression and/or cell proliferation and/or differentiation, as well as further applications derived therefrom.

Proteins influencing cell division, proliferation or differentiation are generally of great interest. These substances open up a variety of possible uses which can be of interest for several applications depending on the specificity of these proteins. Usually, drugs which make use of the effect of suitable proteins to control or prevent pathological situations can also be derived therefrom. In general, newly found proteins which can be produced recombinantly are therefore received with great interest. They do not only have potential pharmaceutical effects themselves but can often also be used as diagnostic means or as means for developing secondary pharmaceutical agents.

With the current systematical elucidation of the sequences of the human genome (human genome project), many sequences are found which obviously code for proteins. In most cases, though, the function of these proteins is completely unknown, so that it cannot be foreseen which possible uses such products might have.

The object underlying the present invention was to detect sequences coding for proteins influencing the cell cycle, cell division and cell proliferation. It was a further object of the present invention to produce corresponding proteins.

In accordance with the present invention this object was accomplished by providing a DNA sequence characterized in that it contains:
(a) a sequence as shown in SEQ ID NO.1 or 2,
(b) a sequence which encodes the same protein as (a) but is degenerate as a result of the genetic code,
(c) a sequence hybridizing under stringent conditions to the sequences of (a) and/or (b),
(d) a genomic sequence containing the sequence of (a), (b) or (c) and further containing one or more introns,
(e) a sequence which differs from (a), (b), (c) or (d) due to its origin from a different species.

In order to find proteins involved in cell cycle activation, a Xenopus oocyte cDNA was prepared and cloned in expression vectors. The primary library was subdivided into pools and plasmid DNA was purified from the pools and in vitro transcribed to obtain mRNAs. The mRNA pools were injected into stage VI oocytes which were incubated to allow for protein expression. Pools which upon microinjection in oocytes were capable of inducing oocyte maturation on their own or of strongly accelerating progesterone-induced maturation were subdivided into smaller pools and reinjected until single positive clones were isolated. Following this approach, out of a huge number of mRNA pools two specific sequences corresponding to SEQ ID NOs.1 and 2 were isolated. These sequences do not correspond by DNA hybridization experiments to any known proteins inducing oocyte maturation, including protein kinase Mos, the protein phosphatase cdc25 and several A and B type cyclins. The mRNA prepared from the two isolated clones containing SEQ ID NO.1 or 2 was used for protein expression and the obtained proteins were found capable of potently inducing oocyte maturation also in the absence of progesterone stimulation. The obtained DNA sequence data as shown in SEQ ID NOs.1 and 2 demonstrate that these two clones contain open reading frames coding for related proteins.

The present invention comprises the sequences shown in SEQ ID NOs.1 and 2 which, however, may also contain certain deviations. In particular, the present invention covers deviations which are present in the DNA only but which, owing to the diversity of the genetic code, encode the same protein as SEQ ID NO. or 2. Furthermore, the present invention comprises sequences which hybridize under stringent conditions with SEQ ID NO. or 2, or sequences deviating therefrom as set out under (b). The present invention also comprehends the corresponding genomic sequences of the cDNA sequences of SEQ ID NO. 1 or 2, or of sequences deviating therefrom as set out under (b) or (c). Such genomic sequences may contain one or more introns which are cleaved off during translation and processing and thus do not influence the finally encoded protein.

Still further, the present invention comprises sequences which deviate from those of (a), (b), (c) or (d) owing to their origin from a different species.

Very often, highly conserved DNAs coding for proteins which have the same activities in different species, such as mouse or human, show only slight differences. In most cases, deviations occur only in some nucleotides and/or in few amino acids of the coded protein. Hence, by means of the concretely disclosed sequences as of SEQ ID NO.1 or 2, corresponding nucleic acids in other species, which code for proteins with the same or a very similar activity, can easily be found. Such similar sequences are therefore comprised by the present invention, too.

The DNA sequences according to the invention encode proteins capable of inducing oocyte maturation and/or promoting cell division, proliferation and/or differentiation.

In a preferred embodiment of the invention the DNA sequences further contain expression controlled sequences which are operably linked to the coding DNA sequence. Any suitable expression control sequences may be used for the present invention. Particularly preferred sequences are those which allow a favourable control of expression, such as sequences allowing induction of expression or inhibition of expression. Induction or inhibition generally takes place via the binding of a respective inductor or inhibitor molecule to operator sequences. Corresponding expression control sequences are known to the person skilled in the art, the lac operator being an example therefor.

A further subject matter of the present invention is an expression vector containing a DNA sequence according to the invention.

As set out above for the DNA sequences, the expression vector also particularly preferably comprises expression control sequences allowing for specific expression control. Also, sequences that allow for positive selection of transformed host cells are known to the man in the art and are preferably introduced in the expression vectors according to the invention.

A further subject matter of the present invention is a protein encoded by a DNA sequence according to the present invention. As explained above, the protein according to the invention, which preferably contains an amino acid sequence according to SEQ ID NO.3 or 4, induces and stimulates cell proliferation and differentiation. Oocyte maturation is also induced by the proteins of the invention. The particularly preferred proteins of SEQ ID NOs.3 and 4 are capable of inducing oocyte maturation considerably faster than the same amount of injected malE-Mos or progesterone treatment. The entire cell cycle in Xenopus oocytes is extraordinarily strongly activated by the proteins of the invention. Only low amounts of the protein of the invention are required to stimulate oocyte maturation, cell proliferation and differentiation.

The protein according to the present invention may have deletions, substitutions and/or additions of amino acids in regions which do not affect the activity. However, the activity of the protein must not be considerably impaired thereby. Further, it is preferred that at the most up to 5 % of the amino acid content of the protein of the invention has deletions, substitutions and/or additions of amino acids. It is not difficult for the skilled artisan to find out which regions may contain deletions, substitutions or additions. Corresponding changes can be made in the nucleic acids, followed by expression and an activity test. By means of site-directed mutagenesis manifold variants can easily be produced and expressed. The person of skill in the art can easily simultaneously test a multitude of such mutants for their activity (high-throughput screening), whereby as a prerequisite at least half the activity of the proteins shown in SEQ ID NO.3 or 4 has to be retained. By means of computer-aided conformation studies the regions of the protein which are less probably involved in the activity of the protein can be determined. Particularly in such regions can mutations be made.

A still further subject matter of the present invention is the use of the protein according to the invention for inducing oocyte maturation and/or promoting cell division, cell proliferation or cell differentiation.

As a further subject matter of the present invention a pharmaceutical composition may be formulated on the basis of this possible use. The pharmaceutical composition according to the invention contains as active agent a protein according to the invention which in particular contains the amino acids as of SEQ ID NO.3 or 4 or sequences derived therefrom which may exhibit the aforementioned mutations, deletions or substitutions.

Preferably, the pharmaceutical composition contains the protein in combination with a pharmaceutically acceptable carrier or adjuvant.

The pharmaceutical composition according to the invention may be used for all pathological situations in which it is desired to promote cell proliferation, cell differentiation or cell maturation. Examples for such applications are the promotion of growth and maturation of specific cell types, e.g. ovarian cells, so that the pharmaceutical composition of the invention is in particular also useful and suitable for fertility treatments.

The protein according to the invention can further be used as diagnostic marker for cell proliferation and/or cell differentiation. The amount of said protein contained in an organism can be correlated to the cell proliferation or differentiation rate. As soon as a basic value has been determined, the amount of this protein present in, e.g., different development stages of cells can be determined, thus showing the particular development status.

A further possible use of the proteins according to the invention lies in their capability of acting as a target for the identification of drugs blocking cell cycle progression and/or cell proliferation and/or cell differentiation. By means of these proteins a multitude of substances can be tested for their blocking and inhibition capability. To this end, a system is provided which comprises cells susceptible to the proteins of the invention, proteins according to the invention and a substance which is to be examined as to its blocking activity. It can then be determined whether the activity of the protein to promote cell proliferation and/or differentiation is weakened or even prevented by said substance. Using high-throughput screening (HTPS), such experiments can be carried out for a multitude of substances simultaneously. A particularly preferable use for identifying substances blocking cell proliferation and/or differentiation lies in the development of drugs for the treatment of cancer or other pathological situations with uncontrolled cell proliferation.

Especially carcinoma grow by uncontrolled cell division, and the inhibition thereof is highly desired. Substances allowing to block such division can be found using the protein of the invention.

Another subject matter of the present invention is the use of the DNA sequences according to the invention as diagnostic marker for the cell proliferation and/or cell differentiation status, whereby the amount of homologous nucleic acids present in the cell is determined by hybridization experiments. Of particular interest is the amount of mRNAs hybridizing to the DNA according to the invention. For this purpose, preferably the DNA sequence according to the invention or a part thereof is labelled, so that after performance of the hybridization experiment the formed double strands may be easily detected. Particularly preferably, a single-stranded DNA sequence should be used corresponding to the antisense strand of the DNA according to the invention. Using such an antisense strand DNA which is complementary to the mRNA, the actual amount of formed protein can be determined on a nucleic acid basis.

In combination with the figures the following examples are to further illustrate the present invention.
- Fig. 1: shows a sequence comparison of proteins Is26 and Is27.
- Fig. 2: shows the result of experiments with injection of small amounts of recombinant maIE-Is26 protein (10 ng) into Xenopus oocytes, leading to oocyte maturation considerably faster than the same amount of injected malE-Mos or a progesterone treatment. Fig. 2 also shows the same experiment where cycloheximide preincubation blocked malE-Mos-induced GVBD (germinal vesicle breakdown) but had no effect on Is26-triggered oocyte maturation.
- Fig. 3: shows the maIE-Is26-induced activation of MAP kinase and cdc2/cyclin B in an immunoblot and by direct measurement of the in vitro kinase activity using MBP and histone H1 as substrates for MAP kinase and cdc2/cyclin.
- Fig. 4: also shows an immunoblot using anti-cdc2 antibodies and an in vitro histone H1 kinase assay.
- Fig. 5: shows a pull-down experiment using rabbit reticulocyte lysates which demonstrates that Is26 can directly bind to B-type cyclins.

### Example 1

To identify novel proteins implicated in cell cycle activation in Xenopus oocytes, an expression cloning strategy was used where a Xenopus oocyte cDNA library was constructed in the FTX5 expression vector. The primary library was subdivided into pools of 150-200 colonies and plasmid DNA was purified from the pools and in vitro transcribed to obtain mRNAs. The mRNA pools were injected into stage VI oocytes which were incubated for 30 - 36 hours to allow protein expression from the injected mRNAs prior to stimulation with progesterone. Finally, those pools which upon microinjection in oocytes were capable either of inducing oocyte maturation on their own or of strongly accelerating progesterone-induced maturation were subdivided into smaller pools and reinjected until single positive clones were isolated.

Using this approach, out of 105 mRNA pools injected in oocytes two clones were isolated which did not correspond by DNA hybridization experiments to proteins that are known to induce oocyte maturation including the protein kinase Mos, the protein phosphatase cdc25 and several A and B type cyclins. The mRNAs prepared from the two isolated clones, which were referred to as Is26 and Is27, were capable of potently inducing oocyte maturation in the absence of progesterone stimulation. DNA sequencing showed that these two clones contained open reading frames that encode for related proteins and were fused in frame to the C-terminus of the myc tag in the FTX5 vector.

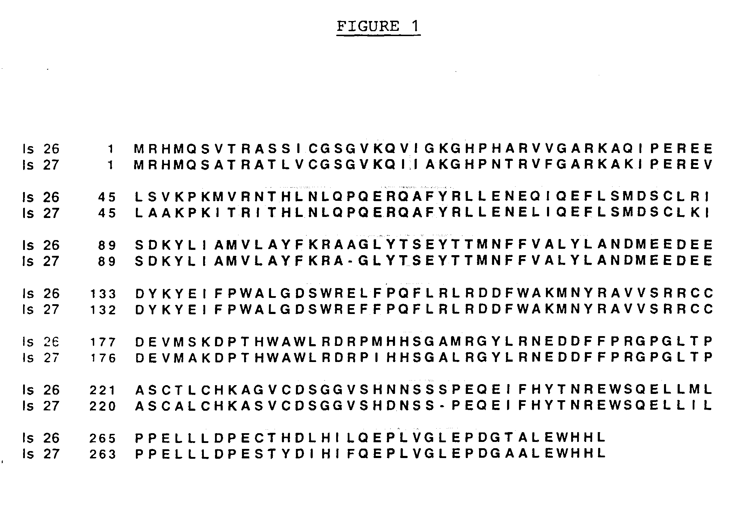

Full-length Is26 and Is27 cDNAs were cloned from a λ ZAP Xenopus oocyte cDNA library using as probes the two cDNAs isolated in the expression screening. The Is26 clone was 1574 base pairs and encoded for a protein of 300 amino acids (SEQ ID NO.1), whilst Is27 was 1357 base pairs in length and encoded for a protein of 298 amino acids (SEQ ID NO.2). Both clones contained stop codons upstream of the first ATG and in the same frame (underlined in SEQ ID NO.1 and 2). The predicted Is26 and Is27 proteins were 91% identical (Fig. 1). When the Is26 and Is27 sequences were tested against DNA and protein sequence data bases, no significant homologies (Blast search) could be detected, suggesting that Is26/Is27 belong to a novel protein family. Programmes were also tested which were designed to identify conserved protein motifs (for example Prosite), but again there was no clue as to the kind of activity that the Is26/Is27 proteins may have. Thus, based on the lack of sequence homology, the Is26/Is27 proteins do not appear to have any known catalytic activity (protein kinase, phosphatase,...)

### Example 2

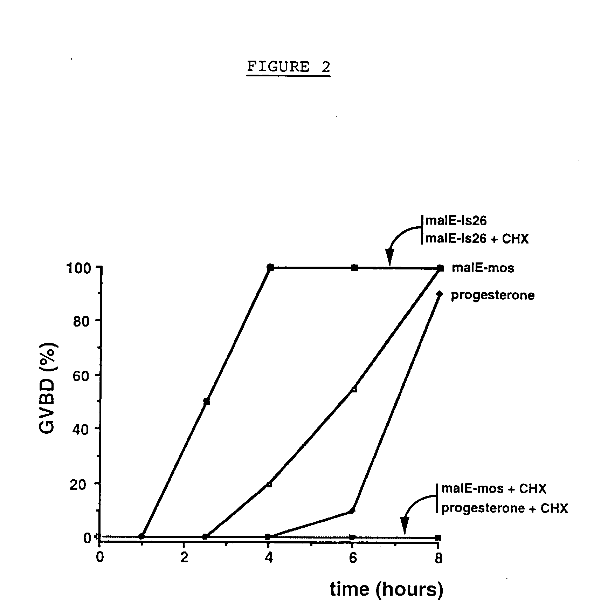

To investigate the function of the Is26/Is27 proteins, the two cDNAs were cloned in the bacterial expression vector pMalc2 downstream of the malE gene. The fusion proteins maIE-Is26 and maIE-Is27 were expressed in and purified from E. coli. Since Is26 and Is27 are very similar in sequence and probably correspond to pseudoalleles, which are quite common in Xenopus, one concentrated on the characterization of Is26 and then confirmed the results obtained using Is27. It was found that the injection into Xenopus oocytes of small amounts of recombinant maIE-Is26 protein (10 ng) was capable of inducing oocyte maturation considerably fater than the same amount of injected malE-Mos or than progesterone treatment (Fig. 2). This experiment using the fusion protein confirmed the results observed with mRNA in vitro transcribed from the Is26 cDNA clone regarding the potency of this novel protein to induce cell cycle activation in Xenopus oocytes. It was also found that injection of only 0.5 ng of maIE-Is26 per oocyte was still acapable of inducing oocyte maturation. The availability of purified malE-ls26 protein also allowed to test the capability of Is26 to induce oocyte maturation in the presence of protein synthesis inhibitors. Preincubation of the oocytes with cycloheximide totally blocked progesterone-induced maturation, consistent with the known essential requirement for translation of maternal mRNAs stored in the oocytes for progesterone to induce maturation. In the same experiment, cycloheximide preincubation also blocked malE-Mos-induced GVBD but it had no effect on Is26-triggered oocyte maturation (Fig. 2).

### Example 3

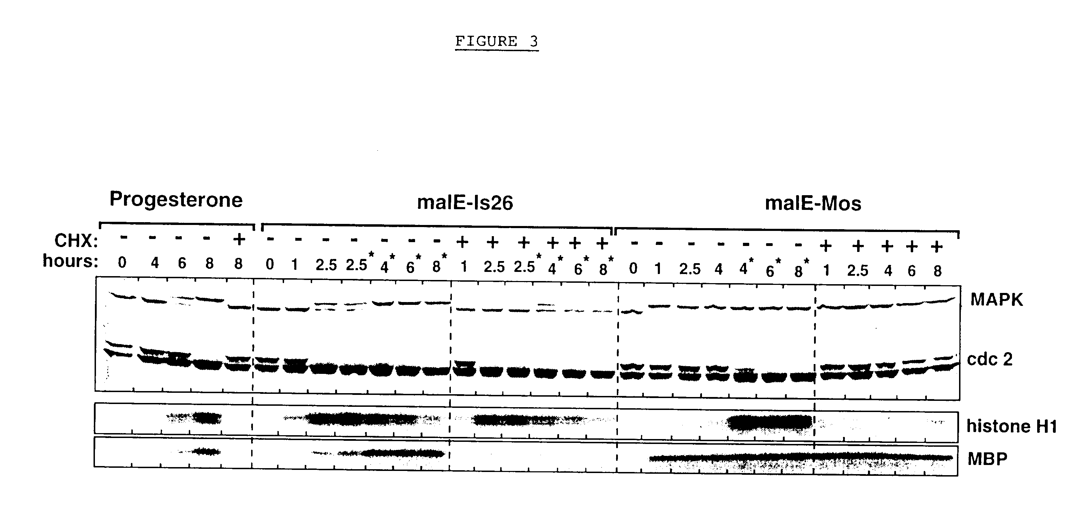

To further characterize the activity of the Is26 protein, the kinetics of activation of MAP kinase (MAPK) and cdc2/cyclin B (MPF) in oocytes induced to mature by malE-ls26 were investigated. MAPK and MPF are normally activated during oocyte maturation and their activation can be detected in oocyte lysates either by immunoblot with anti-MAPK and anti-cdc2 antibodies or by direct measurement of the in vitro kinase activity using MBP and histone H1 as substrates for MAPK and MPF, respectively (Fig. 3). As expected from previous work, we observed that progesterone treatment activates both MAPK and MPF at about the same time, whereas malE-Mos injection activates MAPK well before MPF activation. Interestingly, injection of malE-ls26 rapidly activates MPF somewhat before MAPK. Moreover, MPF appears to be transiently activated by Is26, but the significance of this observation is unclear. In cycloheximide-treated oocytes, the Is26-induced activation of MAPK is very much reduced whereas the activation of MPF is apparently unaffected. This result indicates that the effect of Is26 is more related to MPF activation than to MAPK activation. As expected, cycloheximide totally blocked progesterone-induced activation of both MPF and MAPK, whereas in the case of Mos only MPF but not MAPK activation was compromised by cycloheximide.

### Example 4

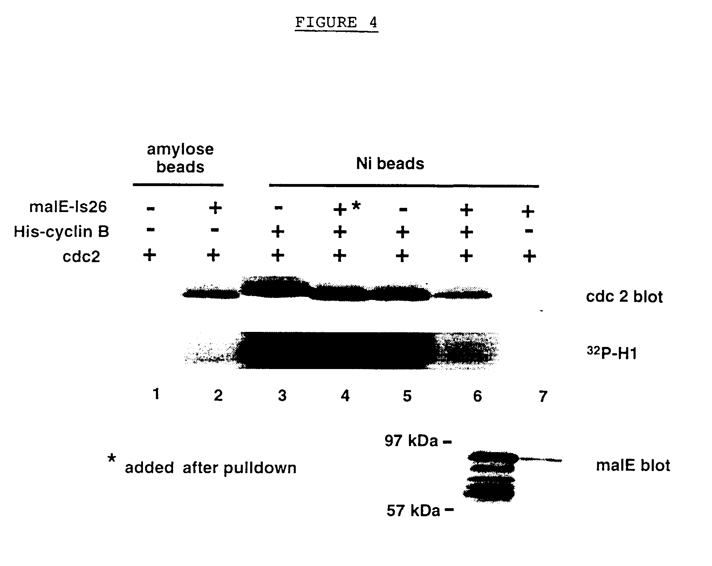

The observation that Is26 can consistently induce oocyte maturation and the activation of MPF independently of new protein synthesis is quite remarkable as only proteins that act very late in the activation pathways, such as cyclins (cdc2 binding and activating subunits) or direct cdc2/cyclinB activators such as the cdc25 phosphatase have been shown to have this strong effect. In order ot address whether Is26 can directly associate with and/or modify the acitvity of cdc2/cyclin B complexes, pull-down experiments were performed. For this purpose, extracts prepared from insect cells infected with cdc2-expressing baculovirus were incubaed with either maIE-Is26 bound to amylose beads or the equivalent amount of cyclin B bound to nickel beads. After extensive washing, the proteins that remained bound to the beads were analyzed by immunoblot using anti-cdc2 antibodies and in vitro histone H1 kinase assay. We found that Is26 bound to cdc2 with almost the same efficiency of cyclin B (Fig. 4). However, while binding of the activating cdc2 subunit cyclin B resulted in high levels of histone H1 kinase activity of the cdc2/cyclin B complexed, ls26 appeared to have little or no effect to stimulate the histone H1 kinase activity of cdc2. When the cyclin B pull-down was done in the presence of a 2-fold molar excess of soluble maIE-Is26, we observed a reduction in the amount of cdc2 bound to cyclin B which also correlated with the expected decrease in the kinase activity of the complexes. This suggests that Is26 may compete with cyclin B to bind to cdc2. By immunoblot using anti-malE antibodies we confirmed that cyclin B was capable of binding Is26 in the presence of cdc2. Our results indicate that Is26 can strongly bind to cdc2 and probably also to cyclin B, but we do not know whether Is26 can complex to cdc2/cyclin B.

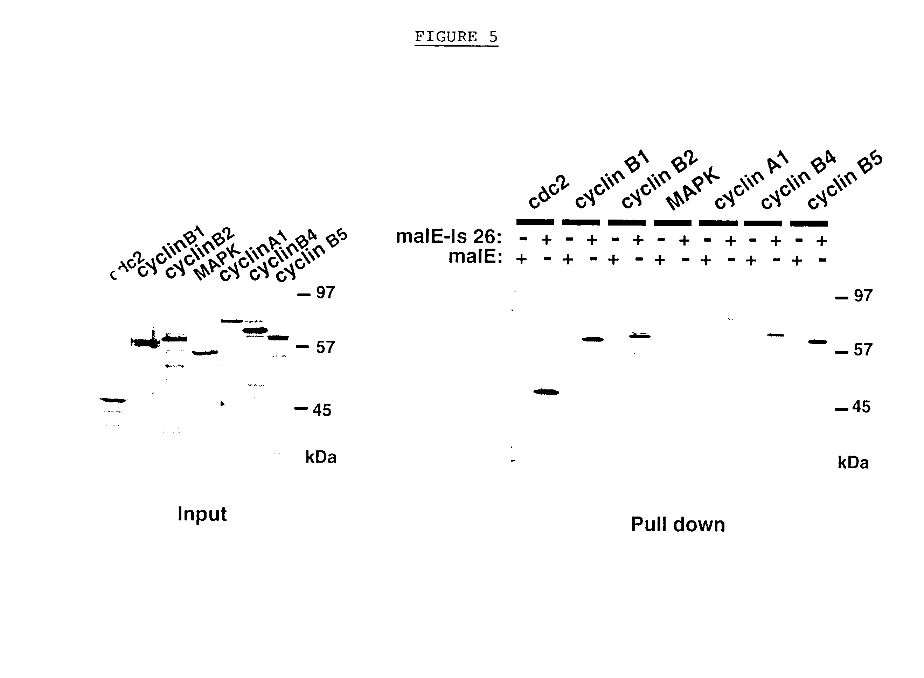

The interaction between Is26 and cdc2 was confirmed using ³⁵5-methionine-labelled cdc2 prepared by coupled transcription/translation in rabbit reticulocyte lysates. We also confirmed in pull-down experiments with rabbit reticulocyte lysates that Is26 can directly bind to B-type cyclins (Fig. 5).

SEQ ID NO. 1

ls 26 cDNA

1 TTA AAC AGG ACT TGC AGC TCC AGT GTA GGT TTT TTC AGA AGC TCC GCC CCA ATG CTG TAT
61 TTT TTT ATT ATT CCA GGA GGC TAT AAA GAG AGC AGA CAA AGG AAG TAG GCG GAG TTC CTG
121 TTT ATC GCC ATT TGG CCA GTG GTG GCT AAG CGC CTG AAG GTG GCT GCT TCC TTT GCT CAG
181 ATC AAC CCT CGG GCC GGT GTC CCC CTT TCT ACA ATG AGG CAC ATG CAG AGT GTA ACC CGG
M R H M Q S V T R 9
241 GCC AGC TCC ATT TGT GGC AGC GGG GTG AAG CAG GTC ATT GGC AAG GGG CAT CCG CAC GCC
A S S I C G S G V K Q V I G K G H P H A 29
301 CGG GTT GTT GGA GCG CGC AAG GCG CAA ATC CCT GAG AGA GAG GAG TTG TCA GTC AAA CCC
R V V G A R K A Q I P E R E E L S V K P 49
361 AAA ATG GTG CGA AAT ACC CAT CTC AAT CTA CAG CCC CAG GAG CGC CAG GCC TTC TAC AGG
K M V R N T H L N L Q P Q E R Q A F Y R 69
421 CTC CTA GAA AAT GAG CAG ATT CAG GAA TTC CTT TCT ATG GAC TCC TGT CTA AGG ATT TCC
L L E N E Q I Q E F L S M D S C L R I S 89
481 GAC AAG TAT CTC ATA GCA ATG GTT CTA GCA TAT TTT AAG CGG GCA GCG GGC CTC TAC ACC
D K Y L I A M V L A Y F K R A A G L Y T 109
541 AGC GAG TAC ACA ACC ATG AAT TTC TTT GTT GCC CTG TAT CTG GCT AAT GAC ATG GAG GAA
S E Y T T M N F F V A L Y L A N D M E E 129
601 GAT GAA GAA GAC TAT AAA TAT GAA ATC TTC CCC TGG GCA CTA GGA GAC TCG TGG CGT GAG
D E E D Y K Y E I F P W A L G D S W R E 149
661 CTT TTC CCA CAA TTT TTG CGT CTC CGG GAC GAC TTC TGG GCT AAA ATG AAC TAC CGA GCA
L F P Q F L R L R D D F W A K M N Y R A 169
721 GTT GTT AgT CGA AGG TGC TGT GAT GAG GTA ATG TCC AAA GAT CCC ACT CAT TGG GCC TGG
V V S R R C C D E V M S K D P T H W A W 189
781 CTG AGA GAT CGC CCC ATG CAT CAC AGC GGG GCC ATG CGT GGT TAC CTT AGA AAC GAG GAC
L R D R P M H H S G A M R G Y L R N E D 209
841 GAC TTT TTC CCC CGG GGT CCA GGC CTT ACA CCA GCC AGC TGT ACA CTT TGC CAT AAA GCA
D F F P R G P G L T P A S C T L C H K A 229
901 GGT GTC TGT GAC TCT GGT GGG GTC TCC CAC AAC AAC TCT TCC TCT CCA GAA CAA GAG ATT
G V C D S G G V S H N N S S S P E Q E I 249
961 TTT CAC TAC ACC AAT AGG GAG TGG TCC CAG GAG CTT CTC ATG TTG CCC CCT GAG CTG TTG
F H Y T N R E W S Q E L L M L P P E L L 269
1021 CTG GAT CCC GAG TGT ACT CAT GAC TTA CAC ATT CTC CAG GAG CCA TTG GTT GGA TTA GAG
L D P E C T H D L H I L Q E P L V G L E 289
1081 CCA GAT GGG ACG GCG CTG GAA TGG CAC CAC CTT TAG TAG CCG ATT GTC TCC TCC GAG CTT
P D G T A L E W H H L * * 300
1141 TTA TTC TTC TCT ACT CAC AAG CTC AGC ACT TAT TCT CTC CTC CTA AGG ACT TGT CAA TGT
1201 TCA GAC TTA ATT GAA ATG GGA GAA GTG AAT ATT CCG ACG GAT GTA GAG CGG GAA TAT GTG
1261 CCC AGA GAA AGT GTT TTG AGT CTG TAT AAA CCG TTG CTT TGT AAA TAA ATA TAT AAA TGT
1321 TCT CTG TGC TGG TCA CTA ATA AAG ATC AGG TAA AAT CAC TTT CAG GTG TAA TTT AAT AGT
1381 ATG TAT GTA GAG TCT TTA ATT CAG CTC TCC ACC AAA TAG TAA CTT GTC ATC ACT GAA CCT
1441 TTG CTT AAC TAC ACT TTT ATT ATT CTG CAC ACA AAT ATT CTG AAG ATC AGA CCG TTC TGT
1501 TTT CAG ATG GGT TGA AAA TAT TAA ACT CAA CAG AAT TCC TGT GGT GTA ATG TAA ATG CAA
1561 AGA TCG ATT AGA CTA

SEQ ID NO. 2

ls 27 cDNA

1 GCT GTA TTT TAC TTT CTT TCA GGA GGC TAT AAA GAC AAC AGA CAG GGG AGG TAG GCA GAG
61 TTC CTG TTC ATC ACC ATT CTT TGG CCG TTG GTG GCT AGG CGC CTG AAG GTG GCT GCT ACC
121 TTT GCT CAG ATC AAC CCT CGG TTC GTT GTC CCC CTT TCT ACA ATG AGG CAT ATG CAG AGT
M R H M Q S 6
181 GCA ACC CGG GCC ACC TTA GTT TGT GGC AGC GGG GTA AAG CAG ATC ATT GCC AAG GGA CAT
A T R A T L V C G S G V K Q I I A K G H 26
241 CCG AAT ACC CGG GTT TTT GGA GCG CGC AAG GCG AAA ATC CCT GAG AGA GAG GTG CTA GCA
P N T R V F G A R K A K I P E R E V L A 46
301 GCC AAA CCC AAG ATC ACG CGC ATT ACA CAT CTC AAT CTA CAA CCC CAG GAG CGC CAG GCC
A K P K I T R I T H L N L Q P Q E R Q A 66
361 TTT TAC AGG CTC CTA GAA AAT GAG CTG ATT CAG GAA TTT CTT TCT ATG GAC TCC TGT CTA
F Y R L L E N E L I Q E F L S M D S C L 86
421 AAG ATT TCA GAC AAG TAT CTC ATA GCA ATG GTT CTA GCA TAT TTT AAG CGG GCG GGC CTC
K I S D K Y L I A M V L A Y F K R A G L 106
481 TAC ACC AGC GAG TAC ACA ACC ATG AAT TTC TTT GTT GCT CTG TAT CTG GCT AAT GAC ATG
Y T S E Y T T M N F F V A L Y L A N D M 126
541 GAG GAA GAT GAA GAA GAC TAT AAA TAT GAA ATC TTC CCC TGG GCA CTA GGA GAT TCA TGG
E E D E E D Y K Y E I F P W A L G D S W 146
601 CGT GAG TTT TTC CCA CAA TTT TTA CGT CTC CGG GAC GAC TTC TGG GCT AAA ATG AAC TAC
R E F F P Q F L R L R D D F W A K M N Y 166
661 CGA GCA GTT GTT AGC CGA AGA TGT TGT GAT GAG GTA ATG GCG AAA GAT CCC ACT CAT TGG
R A V V S R R C C D E V M A K D P T H W 186
721 GCC TGG CTC AGA GAT CGT CCT ATT CAT CAT AGT GGG GCC CTG CGT GGT TAC CTC AGA AAT
A W L R D R P I H H S G A L R G Y L R N 206
781 GAG GAT GAC TTT TTC CCT CGG GGT CCA GGC CTT ACA CCA GCC AGC TGT GCA CTT TGC CAT
E D D F F P R G P G L T P A S C A L C H 226
841 AAA GCA AGT GTC TGT GAC TCT GGT GGG GTG TCC CAT GAC AAC TCT TCT CCA GAA CAA GAG
K A S V C D S G G V S H D N S S P E Q E 246
901 ATT TTT CAC TAC ACC AAT AGG GAG TGG TCC CAG GAA CTT CTC ATC TTG CCA CCT GAA CTG
I F H Y T N R E W S Q E L L I L P P E L 266
961 TTA TTG GAT CCG GAG TCT ACT TAT GAC ATC CAC ATT TTC CAG GAA CCG TTG GTT GGA TTA
L L D P E S T Y D I H I F Q E P L V G L 286
1021 GAG CCA GAT GGG GCA GCC TTG GAA TGG CAC CAC CTT TAG CAC CAT GTC ATC TCT GTG CTT
E P D G A A L E W H H L * 298
1081 TCA TTC TTC TCT AAT CCA CGA GCT CAA GAA GCA CTT AAC CTC TCC TAA GCA CTT GCC CAT
1141 GTC CCT ATT CAG ACT AAT GAA TTA AAT GGG AGA GGT GAC TAT TGC CAT AAA GGG AAG GAT
1201 GCC ACT TAG AGT GGA GAA TAA TAC TTG CCA AAA ATG GTG TTT GGG TCT GTT TAA ACT GTT
1261 GCT ATT TCA GTT GCC TTG TAA ATA AAT AAG TAT AAA AAT GTA TGC TCT GTG CCG GTT GCT
1321 AAT AAA AAA AAA ATC TGG TAT CAA AAA AAA AAA AAA A

SEQ ID NO.3

M R H M Q S V T R 9
A S S I C G S G V K Q V I G K G H P H A 29
R V V G A R K A Q I P E R E E L S V K P 49
K M V R N T H L N L Q P Q E R Q A F Y R 69
L L E N E Q I Q E F L S M D S C L R I S 89
D K Y L I A M V L A Y F K R A A G L Y T 109
S E Y T T M N F F V A L Y L A N D M E E 129
D E E D Y K Y E I F P W A L G D S W R E 149
L F P Q F L R L R D D F W A K M N Y R A 169
V V S R R C C D E V M S K D P T H W A W 189
L R D R P M H H S G A M R G Y L R N E D 209
D F F P R G P G L T P A S C T L C H K A 229
G V C D S G G V S H N N S S S P E Q E I 249
F H Y T N R E W S Q E L L M L P P E L L 269
L D P E C T H D L H I L Q E P L V G L E 289
P D G T A L E W H H L * * 300

SEQ ID NO. 4

M R H M Q S 6
A T R A T L V C G S G V K Q I I A K G H 26
P N T R V F G A R K A K I P E R E V L A 46
A K P K I T R I T H L N L Q P Q E R Q A 66
F Y R L L E N E L I Q E F L S M D S C L 86
K I S D K Y L I A M V L A Y F K R A G L 106
Y T S E Y T T M N F F V A L Y L A N D M 126
E E D E E D Y K Y E I F P W A L G D S W 146
R E F F P Q F L R L R D D F W A K M N Y 166
R A V V S R R C C D E V M A K D P T H W 186
A W L R D R P I H H S G A L R G Y L R N 206
E D D F F P R G P G L T P A S C A L C H 226
K A S V C D S G G V S H D N S S P E Q E 246
I F H Y T N R E W S Q E L L I L P P E L 266
L L D P E S T Y D I H I F Q E P L V G L 286
E P D G A A L E W H H L * 298

## Claims

1. A DNA sequence,
**characterized** in that it contains:
(a) a sequence as shown in SEQ ID NO.1 or 2,
(b) a sequence which encodes the same protein as (a) but is degenerate as a result of the genetic code,
(c) a sequence hybridizing under stringent conditions to the sequences of (a) and/or (b),
(d) a genomic sequence containing the sequence of (a), (b) or (c) and further containing one or more introns,
(e) a sequence which differs from (a), (b), (c) or (d) due to its origin from a different species.

2. A DNA sequence according to claim 1,
wherein it encodes a protein that is capable of inducing oocyte maturation and/or promoting cell division.

3. A DNA sequence according to claim 1 or 2,
**characterized** in that it further contains expression control sequences operably linked to the coding DNA sequence.

4. Expression vector,
**characterized** in that it contains a DNA sequence according to anyone of claims 1 to 3.

5. Protein
**characterized** in that it is encoded by a DNA sequence according to anyone of claims 1 to 3.

6. Protein according to claim 5,
**characterized** in that it contains an amino acid as shown in SEQ ID NO.3 or 4.

7. Protein according to claim 5 or 6,
**characterized** in that it shows an oocyte maturation inducing activity and/or a cell division promoting activity.

8. Protein according to anyone of claims 5 to 7,
**characterized** in that it contains deletions, substitutions and/or additions of amino acids that do not substantially affect its activity.

9. Protein according to anyone of claims 5 to 8,
wherein a second protein is fused to build a fusion protein.

10. Use of a protein according to anyone of claims 5 to 9 for inducing oocyte maturation and/or promoting cell division and/or differentiation and/or proliferation.

11. Pharmaceutical composition containing as active agent a protein according to anyone of claims 5 to 9.

12. Pharmaceutical composition according to claim 11, containing the protein in combination with a pharmaceutically acceptable carrier or adjuvant.

13. Use of a pharmaceutical composition according to claim 10 or 11 for promoting cell proliferation, cell differentiation, or for fertility treatments.

14. Use of a protein according to anyone of claims 5 to 9 as a diagnostic marker for cell proliferation and/or cell differentiation.

15. Use of a protein according to claims 5 to 9 as a target for the identification of drugs that block cell cycle progression and/or cell proliferation and/or cell differentiation.

16. Use according to claim 15 for the development of pharmaceuticals for the treatment of cancer or other pathological situations with uncontrolled cell proliferation.

17. Use of a DNA sequence according to anyone of claims 1 to 3 or a part thereof as diagnostic marker for cell proliferation and/or cell differentiation for hybridization expriments to determine the amount of homologous nucleic acid sequences.
